(19) Europäisches Patentamt
European Patent Office
Office européen des brevets

(11) EP 0 319 986 B1

(12) # EUROPEAN PATENT SPECIFICATION

(45) Date of publication and mention of the grant of the patent: **12.03.1997 Bulletin 1997/11**

(51) Int. Cl.[6]: **A61K 31/445**, A61K 31/70, A61K 31/435

(21) Application number: **88120564.5**

(22) Date of filing: **08.12.1988**

(54) **Anti-plasmin depressant**

Anti-Plasmin Depressant

Dépressant anti-plasmin

(84) Designated Contracting States:
**AT DE IT NL**

(30) Priority: **09.12.1987 JP 311348/87**

(43) Date of publication of application:
**14.06.1989 Bulletin 1989/24**

(73) Proprietor: **NIPPON SHINYAKU COMPANY, LIMITED**
**Minami-ku Kyoto-shi Kyoto 601 (JP)**

(72) Inventors:
- **Yoshikuni, Yoshiaki**
  **Uji-shi Kyoto-fu 611 (JP)**
- **Ojima, Nobutoshi**
  **Moriyama-shi Shiga-ken 524 (JP)**
- **Mori, Kazuya**
  **Nakakyo-ku Kyoto-shi 604 (JP)**

(74) Representative: **Vogeser, Werner, Dipl.-Ing.**
**Patent- und Rechtsanwälte**
**Hansmann, Vogeser, Dr. Boecker,**
**Alber, Dr. Strych, Liedl**
**Albert-Rosshaupter-Strasse 65**
**81369 München (DE)**

(56) References cited:
**EP-A- 0 022 192**
**WO-A-87/03903**
**GB-A- 2 020 278**

- **J. PHARMACOBIO-DYN., vol. 11, no. 5, May 1988, pages 356-362; Y. YOSHIKUNI et al.: "Inhibition of intestinal alpha-glucosidase and postprandial hyperglycemia by N-substituted moranoline derivatives"**
- **PATENT ABSTRACTS OF JAPAN, vol. 10, no. 94 (C-338)[2151], 11th April 1986; & JP-A-60 224 675 (MEIJI SEIKA K.K.) 09-11-1985**
- **Decision making in Drug Research, 1983, p.173-188**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 319 986 B1

## Description

The present invention relates to the use of a composition, comprising as the main ingredient a compound selected from the group consisting of (1) through (4) described below:

(1) a compound represented by the following general formula [II] and a physiologically acceptable acid addition salt thereof. as well as a quaternary salt thereof;
(2) nojirimycin and a physiologically acceptable acid addition salt thereof;
(3) 1,4-bis(3-moranolino-1-propenyl)benzene and a physiologically acceptable acid addition salt thereof; and,
(4) castanospermine;

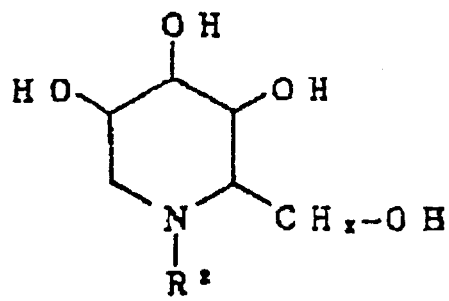

[II]

wherein $R^2$ represents a hydrogen atom or an amino, methoxyethyl, methoxyethoxyethyl, 2-hydroxyhexadecyl, 3-carbamoylpropyl, undecyl, 2-hydroxytetradecyl or 3-carboxy-2-propenyl group, or an alkyl, carboxyalkyl, alkyloxycarbonylalkyl, hydroxyalkyl, oycloalkylalkyl, arylalkyl, aryloxyalkyl, alkenyl, hydroxyalkenyl, arylalkenyl, aryloxyalkenyl or alkylcarbamoylalkyl; 3-fluoro-4-methylphenylbutyl, 2-hydroxy-3-phenoxypropyl, 3-o-chlorophenoxypropyl, p-ethoxycarbonylphenoxyethyl, 2-hydroxy-3-tolyloxypropyl, 2-hydroxy-3-p-methoxyphenoxypropyl, 2-hydroxy-3-p-chlorophenoxypropyl, 3-(m-methoxyethoxyphenyl)-2-butenyl, γ-methyl-4-bromocinnamyl, p-ethoxycinnamyl, p-isopropyloxycinnamyl, γ-methyl-m-methylcinnamyl, 4-(m-methoxyphenyl)-3-pentenyl, 4,4-diphenyl-3-butenyl, 3-(p-chlorophenyl)-2-butenyl, 3-(p-methylphenyl)-2-butenyl, 4-(p-chlorophenyl)-3-pentenyl, 4-(m-chlorophenyl)-3-pentenyl, 4-(o-chlorophenyl)-3-pentenyl, 4-(p-phenoxyphenyl)-3-pentenyl, 4-(p-ethoxyphenyl)-3-pentenyl, m-methoxycinnamyl, 3-(m-chlorophenyl)-2-butenyl, 4-(p-chlorophenyl)-3-butenyl, p-carboxycinnamyl, m-trimethylammonioethoxycinnamyl, p-trimethylammonioethoxycinnamyl; wherein the cycloalkyl of the cycloalkylalkyl is cyclopropyl, cyclobutyl or cyclopentyl; the aryl is phenyl or naphthyl; the alkyl groups contain up to 10 carbon atoms,

for the manufacture of a medicament for the treatment of myocardial infarction, cerebral infarction or for the prevention of postoperative thrombus or thrombosis.

For formation of hemostatic thrombus caused in the case of suffering physical damage in blood vessel, aggregation of platelet and subsequent precipitation of fibrin are inevitably required. On the other hand, thrombus inhibits blood flow in blood vessel to cause ischemia or necrosis of tissue, resulting in myocardial infarction or cerebral infarction. Therefore, the living body is prepared with a mechanism for removing an excess of thrombus in blood vessel, where fibrinolysis by the plasminogen-plasmin system takes a great role in the mechanism.

Plasminogen is activated with a plasminogen activator to convert into plasmin and plasmin decomposes fibrin (fibrinolysis). Abnormality in this mechanism causes diseases such as myocardial infarction, cerebral infarction, etc.

Various causes for these modern diseases are considered but upon treatment of these diseases, fibrinolytic therapy in which the formed thrombus is lysed to improve an ischemic state of tissue is effective. For this purpose, plasminogen activators such as urokinase, streptokinase, etc. have been administered. Therefore, there is a possibility that a substance for accelerating secretion of urokinase would be a good drug in the therapy of thrombolysis described above.

The living body is also prepared with a mechanism for protecting from excessive fibrinolysis. It is known that for example, $\alpha_2$-plasmin inhibitor (hereafter referred to as "$\alpha_2$-PI") which is a plasmin inhibitor instantaneously inhibits a plasmin activity to inhibit fibrinolysis. Therefore, $\alpha_2$-PI acts as a treatment inhibitor in therapy of fibrinolysis and is also formed as a sort of protein in the acute phase to become one of causes for postoperative thrombus.

From J. Pharmacobio-Dyn., 11, (1988) the inhibition of intestinal alpha-glucosidase and postprandial hyperglycemia by N-substituted moranoline derivatives is known.

WO-A-87/03903 relates to the therapeutic use of processing glucosidase inhibitors to regulate the replication, infection and pathogenesis of animal retroviruses such as the aetiological agents of human acquired immune deficiency

syndrome, feline leukemia, equine infectious anemia and chronic lentiviral diseases.

GB-A-2 020 278 refers to N-substituted moranoline derivatives of a specific formula which are considered as being useful for the treatment of hyperglycemia and related diseases.

Patent Abstracts of Japan, volume 10, No. 94, (C-338)[2151], 11 April 1986, referring to JP-A-60224675, relates to a novel 1-deoxynojirimycin derivative and a hypolipemic containing the same which should be used in hyperlipemia, arteriosclerosis and a variety of diseases caused thereby.

EP-A-0 022 192 relates to 1-alkadiene-2,4-yl-hydroxymethyl-3,4,5-trihydroxy-piperidine, a process for the production of said compound as well as its use as pharmaceuticals, especially the inhibitory effect on alpha-glucoside hydrolases and lipid absorption.

From the foregoing, it is suggested that a drug for lowering the activity of $\alpha_2$-PI would more enhance the effect in therapy of fibrinolysis and would serve in preventing postoperative thrombus or thrombosis.

Therefore, an object of the present invention is to provide an excellent $\alpha_2$-PI inhibitor for producing drugs effective for treatments of myocardial infarction or cerebral infarction or for the prevention of postoperative thrombus or thrombosis.

It is also an important object of the present invention to provide the use of a substance for accelerating secretion of urokinase since urokinase is a plasminogen activator.

In order to achieve the objects described above, the present inventors have screened many groups of compounds and as a result of extremely lucky chances, they have clarified that the compounds in accordance with the present invention possess an excellent $\alpha_2$-PI depressing activity and urokinase secretion accelerating activity and, at the same time, they have found that the compounds also accelerate the fibrinolysis accelerating effect in exhibiting the fibrinolytic effect and, could have reached the present invention.

As will be Later described, the compounds of the present invention exhibit an excellent $\alpha_2$-PI depressing activity and urokinase secretion accelerating activity and can be used as drugs for treating myocardial infarction or cerebral infarction as fibrinolytic agents so that the compounds are extremely useful.

The compounds used in the present invention have structural characteristics as recited in the claims.

As $R^1$ and $R^2$ in general formulae [I] and [II], mention may be made of the substituents described above.

The term alkyl as used herein refers to an alkyl group having 1 to about 10 carbon atoms; a lower alkyl, for example, methyl, ethyl, are preferred.

As the carboxyalkyl, mention may be made of, for example, carboxymethyl, carboxyethyl, carboxypropyl. The alkyl having a larger carbon atom number is also included in the compounds of the present invention.

As the alkyloxycarbonylalkyl, mention may be made of, for example, methoxycarbonylmethyl, methoxycarbonylethyl, methoxycarbonylpropyl, methoxycarbonylbutyl, ethoxycarbonylmethyl, ethoxycarbonylethyl, ethoxycarbonylpropyl, ethoxycarbonylbutyl. The alkyloxycarbonylalkyl having a larger carbon atom number in the alkyl is also included in the compounds of the present invention.

As the hydroxyalkyl, mention may be made of, for example, hydroxymethyl, hydroxyethyl, hydroxypropyl, hydroxybutyl. The hydroxyalkyl having a larger carbon atom number in the alkyl is also included in the present invention.

As the cycloalkylalkyl, mention may be made of, for example, cyclopropylmethyl, cyclobutylmethyl, cyclopentylmethyl. The cycloalkylalkyl having a larger carbon atom number in the alkyl is also included in the present invention.

As the arylalkyl, mention may be made of, for example, benzyl, phenethyl, phenylpropyl, phenylbutyl, phenylpentyl, naphtylmethyl, naphtylethyl, naphtylpropyl, naphtylbutyl. The arylalkyl having a larger carbon atom number in the alkyl is also included in the present invention.

As the aryloxyalkyl, mention may be made of, for example, phenoxymethyl, phenoxyethyl, phenoxypropyl, phenoxybutyl, naphtyloxymethyl, naphtyloxyethyl, naphtyloxypropyl, naphtyloxybutyl. The aryloxyalkyl having a larger carbon atom number in the alkyl is also included in the present invention.

As the alkenyl, mention may be made of, for example, vinyl, propenyl, butenyl. The alkenyl having a larger carbon atom number is also included in the present invention.

As the hydroxyalkenyl, mention may be made of, for example, hydroxyvinyl, hydroxypropenyl, hydroxybutenyl. The hydroxyalkenyl having a larger carbon atom number is also included in the present invention.

As the arylalkenyl, mention may be made of, for example, phenylvinyl, phenylpropenyl, phenylbutenyl, naphthylvinyl, naphthylpropenyl, naphthylbutenyl. The arylalkenyl having a larger carbon atom number is also included in the present invention.

As the compounds used in the present invention, mention may be made of, in addition to those described above, nojirimycin obtained by replacing OH at the 1-position of the basic structure of moranoline and derivatives thereof (for example, N-substituted derivatives). A group of these compounds also exhibit similar activities to that of the moranoline derivatives (N-substituted derivatives) and are included in the compounds used in the present invention.

Compounds having the moranoline basic structure as bis-form are also included in the present invention. These compounds also exhibit similar pharmacological effect to that of the other compounds used in the present invention.

Representative examples of these compounds used in accordance with the present invention include nojirimycin and pharmacologically acceptable salts thereof, 1,4-bis-(3-moranolino-1-propenyl)benzene and pharmacologically

acceptable salts thereof. These compounds also exhibit an excellent $\alpha_2$-PI lowering activity and urokinase secretion accelerating activity, as in the other compounds used in the present invention.

The compounds used in the present invention can be prepared in a conventional manner. A process for preparing, for example, N-butylmoranoline is exemplified below.

[Preparation Example 1]

Moranoline, 50 g, 126 g of n-butyl bromide and 170 g of potassium carbonate were added to 1300 ml of dimethylformamide. The mixture was stirred at room temperature for 7 days to complete the reaction. After impurities were removed by filtration, the solvent was distilled off under reduced pressure and 1000 ml of a strongly acidic ion exchange resin Dowex 50W x 2 ($H^+$) was passed. After thoroughly washing with water, elution was performed with 1N ammonia water. The eluate was concentrated under reduced pressure. Thereafter, 50 ml of methanol was added to the concentrate. The mixture was allowed to stand at room temperature and the formed crystals (47 g) were collected.

After the crystals were dissolved in 500 ml of methanol with heating, the solution was cooled to room temperature and then treated with activated charcoal. After concentrating to about 100 ml, the concentrate was allowed to stand at room temperature and 40 g of crystals precipitated were collected. After the crystals were dissolved in 200 ml of methanol with heating, the solution was gently concentrated. The concentrate was allowed to stand at room temperature and crystals precipitated were collected. The crystals were thoroughly dried at 70°C under reduced pressure to give 34 g of objective N-(n-butyl)moranoline. Yield, 50.6%; melting point, 128-129°C.

| Elemental analysis: | | | |
|---|---|---|---|
| Calcd. (%) | C: 54.78 | H: 9.65 | N: 6.39 |
| Found (%) | C: 54.57 | H: 9.65 | N: 6.60 |

$[\alpha]_D^{24}$ -14.59 (1%, water)
$^1$H-NMR: 0.88 (3H, t, J=7.2Hz, $\underline{CH_3}CH_2CH_2CH_2$-), 1.16-1.56 (4H, m, J=7.2Hz, $CH_3\underline{CH_2}\underline{CH_2}CH_2$-), 2.17-2.36 (2H, m, J=7.2Hz, $CH_3CH_2CH_2\underline{CH_2}$-), 2.48-2.82 (2H, m, H-1a, H-5), 3.02 (1H, dd, J=5.1, 11.4Hz, H-4), 3.22 (1H, t, J=9.0Hz, H-4), 3.66 (1H, t, J=9.4Hz, H-4), 3.44-3.6 (1H, m, H-2), 3.74-3.96 (H x 2, dd x 2, $H_6$, $H_{6'}$)

[Preparation Example 2]

Moranoline, 5 g, 13 g of n-butyl bromide and 17 g of potassium carbonate were added to 130 ml of dimethylformamide. The mixture was reacted at 100°C for 5 hours. Then, the reaction mixture was treated in a manner similar to Preparation Example 1 to give 5.1 g of N-(n-butyl)moranoline. Yield, 75.8%.

[Preparation Example 3]

To 100 ml of methanol was added 5 g of moranoline. While stirring at room temperature, a solution of 20 ml of n-butylaldehyde in 50 ml of methanol having dissolved therein 0.7 g of hydrogen chloride and 3 g of $NaCNCH_3$ were added to the mixture. The reaction was carried out overnight. After completion of the reaction, the solvent was removed under reduced pressure. The residue was dissolved in followed by partition with chloroform. The aqueous phase was passed through a column of 200 ml Diaion SA-11A ($OH^-$) type followed by thorough washing with water. The passing liquid was combined with the washing liquid. The mixture was passed through a column of 200 ml Dowex 50W x 28 ($H^+$) type. After thoroughly washing, elution was conducted with 1N ammonia water. After the solvent was distilled off under reduced pressure, the eluate was crystallized from ethanol. Recrystallization from ethanol gave 5.1 g of N-(n-butyl)moranoline. Yield, 75.8%.

Typical examples of the compounds in accordance with the present invention include the following compounds.

Compound No. 1 Moranoline
Compound No. 2 N-methylmoranoline
Compound No. 2a N-(n-Butyl)moranoline
Compound No. 3 N-5-Methoxycarbonylpentylmoranoline tosylate
Compound No. 4 N-Hydroxyethylmoranoline
Compound No. 5 (N-Methoxycarbonylbutyl)moranoline

Compound No. 6 Nojirimycin bisulfite
Compound No. 7 1,4-Bis-(3-moranolino-1-propenyl)benzene dihydrochloride
Compound No. 8 N-Hexylmoranoline tosylate
Compound No. 9 N-Isoprenylmoranoline
Compound No. 10 N-(2-Hydroxydecyl)moranoline tosylate
Compound No. 11 N-10-Carboxydecylmoranoline sodium salt
Compound No. 12 N-(3-Phenylpropyl)moranoline tosylate
Compound No. 13 N-Benzylmoranoline tosylate
Compound No. 14 N-Cinnamylmoranoline hydrochloride
Compound No. 15 N-4-Phenylbutylmoranoline tosylate
Compound No. 16 N-(2-Phenoxyethyl)moranoline
Compound No. 17 N-(3-Phenoxypropyl)moranoline tosylate
Compound No. 18 N-5-(Phenylpentyl)moranoline tosylate
Compound No. 19 N-(2-Cyclopentylethyl)moranoline tosylate
Compound No. 20 N-[3-(3-Methoxyethoxyphenyl)-2-butenyl]moranoline
Compound No. 21 N,N-Dimethylmoranoline ammonium iodide
Compound No. 22 N-Ethylmoranoline
Compound No. 23 N-Cinnamylmoranoline
Compound No. 24 N-Geranylmoranoline tosylate
Compound No. 25 N-(2-Hydroxy-3-phenoxypropyl)moranoline tosylate
Compound No. 26 N-Farnesylmoranoline tosylate
Compound No. 27 N-10-(N-Methylcarbamoyl)decylmoranoline
Compound No. 28 N-(4-Phenyl-3-butenyl)moranoline tosylate
Compound No. 29 N-(3-Phenyl-2-methyl-2-propenyl)moranoline
Compound No. 30 N-(3-o-Chlorophenoxypropyl)moranoline
Compound No. 31 N-(γ-Methyl-4-bromocinnamyl)moranoline
Compound No. 32 N-[4-(3-Fluoro-4-methylphenyl)butyl]moranoline
Compound No. 33 N-(p-Ethoxycinnamyl)moranoline
Compound No. 34 N-(p-Isopropoxycinnamyl)moranoline
Compound No. 35 N-(γ-Methyl-m-methylcimnnamyl)moranoline
Compound No. 36 N-(4-m-Methoxyphenyl-3-pentenyl)moranoline
Compound No. 37 N-(p-Ethoxycarbonylphenoxyethyl)moranoline[emiglitate]
Compound No. 38 Castanospermine

In addition to those described above, the compounds used in accordance with the present invention further include the following compounds.

N-Isobutylmoranoline tosylate
N-Hydroxyethylmoranoline tosylate
N-Aminomoranoline hydrobromide
N-Methoxyethylmoranoline tosylate
N-Methoxyethoxyethylmoranoline tosylate
N-Decylmoranoline tosylate
N-(2-Hydroxyhexadecyl)moranoline tosylate
N-(2-Hydroxy-3-p-tolyloxypropyl)moranoline tosylate
N-(2-Hydroxy-3-p-methoxyphenyloxypropyl)moranoline tosylate
N-(2-Hydroxy-3-p-chlorophenyloxypropyl)moranoline tosylate
N-3-Carbamoylpropylmoranoline
N-Nonylmoranoline tosylate
N-Undecylmoranoline tosylate
N-(2-Hydroxytetradecyl)moranoline tosylate
N-(4,4-Diphenyl-3-butenyl)moranoline
N-5-Carboxypentylmoranoline
N-farnesylmoranoline
N-(γ-Methyl-4-chlorocinnamyl)moranoline
N-(γ-Methyl-4-methylcinnamyl)moranoline
N-(4-p-Chlorophenyl-3-pentenyl)moranoline
N-(4-m-Chlorophenyl-3-pentenyl)moranoline
N-(4-o-Chlorophenyl-3-pentenyl)moranoline

N-(4-p-Phenoxyphenyl-3-pentanyl)moranoline
N-(4-p-Ethoxyphenyl-3-pentenyl)moranoline
N-(m-Methoxycinnamyl)moranoline
N-[3-(3-Chlorophenyl)-2-butenyl]moranoline
N-[4-(4-Chlorophenyl)-3-butenyl]moranoline
N-(4-Carboxylcinnamyl)moranoline hydrochloride
N-(3-Carboxy-2-propenyl)moranoline
N-(m-Triethylammonioethoxycinnamyl)moranoline dipicrate
N-Isopropylmoranoline
N-(p-Trimethylammonioethoxycinnamyl)moranoline chloride hydrochloride

In case that the compounds used in the present invention are administered as medical drugs, the compounds are administered to animals including human as they are or as medical compositions containing, for example, 0.1 to 99.5%, preferably 0.5 to 90% in a pharmaceutically acceptable non-toxic and inert carrier.

As the carrier, at least one of a solid, semi-solid or liquid diluent, filler and other aid for formulation is used. The pharmaceutical composition is desirably administered in a dosage unit mode. The pharmaceutical composition can be administered orally, or into tissue such as intravenously, topically (subcutaneously) or intrarectally. The pharmaceutical composition is, needless to say, administered in a preparatory form suited for these administration modes. Oral administration is particularly preferred.

Dosage as a fibrinolytic agent is controlled desirably depending upon conditions of the patient such as age, body weight, route for administration, properties and degree of disease but the composition is generally administered to adult as the effective ingredient in a daily dose ranging from 50 to 3090 mg/person, preferably from 500 to 1000 mg/person. Depending upon case, the lower dose is also effective and conversely, a larger dose may be required. It is also desired to administer in portions twice or thrice a day.

[Examples]

Hereafter the $\alpha_2$-PI lowering activity the urokinase secretion accelerating activity as well as and toxicity of the compounds of the present invention are described in detail and the present invention is described below in more detail.

Test Example 1 Activity in vitro

It is known that human hepatic cancer-derived HepG2 cells synthesize and secrete $\alpha_2$-plasmin inhibitor ($\alpha_2$-PI). The HepG2 cells, 2 x $10^6$, were inoculated on a plastic culture plate (diameter of 100 mm) made by Falcon Inc. and cultured in Eagle's minimum medium containing 10% bovine fetal serum.

Three days after, cells adhered to the bottom of the plate were washed twice with Dulbecco's phosphate buffer followed by culturing in 8 ml of serum-free Eagle's medium (containing no Phenol Red) containing 200 µg/ml of a specimen of the compound of the present invention for further 3 to 4 days. After culture, 7 ml of the medium was collected and concentrated to about 1 ml using Centriflow (CF25) made by Falcon Inc. The concentrate was further freeze dried.

To the freeze dried sample, 0.7 ml of 50 mM Tris buffer containing 8.1 mg/ml of monomethylamine hydrochloride was added to dissolve therein, whereby the solution was concentrated to 10-fold.

To 100 µl of the concentrated sample, 50 µl of 15 mCU plasmin solution was added and further 50 µl of 0.25 µmole S-2251 synthetic chromogenic substrate solution was added thereto followed by reacting at 37°C for 10 minutes. By adding 1 ml of 2% citrate solution, the reaction was stopped. p-Nitroanilide released from the S-2251 substrate was measured at O.D. of 405 nm.

A sample in which decomposition of the S-2251 substrate was measured using the aforesaid Tris buffer instead of the concentrated sample was made a control showing 100% plasmin activity and the concentrated sample cultured without adding a test compound was made a control showing 100% $\alpha_2$-PI activity. The results were calculated according to the following equation.

$$\alpha_2\text{-PI Activity (\%)} = \frac{A_1 - A_2}{A_1 - A_3} \times 100$$

wherein $A_1$, $A_2$ and $A_3$ represent absorbance in 100% control, absorbance when the concentrated sample was used and absorbance in $\alpha_2$-PI activity 100% control, respectively. The numbed of the samples was 3, respectively. The results are shown in Table 1. It is evident that the used compounds can lower the $\alpha_2$-PI activity.

Table 1

| Sample Compound No. | $\alpha_2$-PI Activity (%) | Sample Compound No. | $\alpha_2$-PI Activity (%) |
|---|---|---|---|
| Control | 100 | 11 | 88 |
| 1 | 63 | 12 | 50 |
| 2 | 62 | 13 | 80 |
| 2a | 59 | 14 | 51 |
| 3 | 70 | 15 | 39 |
| 4 | 70 | 16 | 74 |
| 5 | 79 | 17 | 65 |
| 6 | 67 | 18 | 20 |
| 7 | 86 | 19 | 63 |
| 8 | 56 | 20 | 37 |
| 9 | 55 | | |

Test Example 2 Activity in vivo

Three (3) male Beagle dogs were used for the control group and the administered group, respectively, as animals to be administered.

A test sample (Compound No. 2) was dissolved in a concentration of 10 mg 0.1 ml, using 0.1 M phosphage buffer (pH 7.2). After dissolution, the solution was sterilized by filtration through a sterile filter (pore size, 0.2 μm) and then 0.3 ml of the solution was administered per 1 kg of body weight (30 mg/kg). The administration was made through the right front limb vein for consecutive 7 days. Collected blood was mixed with 3.8% sodium citrate in a ratio of 1 : 9 by volume. By centrifugation at 3000 rpm for 15 minutes, plasma was isolated.

The $\alpha_2$-PI activity was measured as an inhibition activity against decomposition of plasmin with the synthetic chromogenic substrate S-2251. The results of measurement are shown as change in inhibition activity after administration based on 100% of the plasmin inhibition activity prior to administration of the test sample (Table 2). The sample number in each group was 3 samples.

The $\alpha_2$-PI activity was obviously depressed by consecutive administration of the test compound in a dose of 30 mg/kg.

Table 2

| [$\alpha_2$-PI activity (%)] | | | | | | | | |
|---|---|---|---|---|---|---|---|---|
| Day | 0 | 1 | 2 | 3 | 4 | 5 | 6 | 7 |
| C | 100 | 98 | 92 | 94 | 96 | 98 | 100 | 106 |
| Sample | 100 | 95 | 83 | 84 | 76 | 75 | 86 | 89 |

C : control
Sample: test sample

Test Example 3 Test on fibrinolysis in vitro

A test sample (Compound No. 2) was consecutively administered to Beagle dogs in a dose of 30 mg/kg once a day.

Plasma was isolated prior to and 4 days after the administration. The $\alpha_2$-PI activity in plasma was measured and at the same time, fibrin clot was formed in vitro using the plasma. When urokinase as a thrombolytic agent was acted on the fibrin clot, a degree of lysis was compared between plasma prior to the administration and plasma 4 days after the administration.

To 300 µl of the isolated plasma, 40 µl of $^{125}$I-fibrinogen (0.1 mci/ml) was added and 50 µl each of the mixture was dispensed in a test tube. 5 µl each of a solution mixture of 25 U/ml thrombin and 0.5 M calcium chloride was added to each test tube, which was incubated at 37°C for 30 minutes to prepare fibrin clots. Solutions of 15 and 30 U/ml of urokinase in 2% albumin solution and the resulting solution ware charged in each test tube by 1 ml each.

After incubating at 37°C for 12 hours, 25 µl of the supernatant was collected in an RAI tube and $^{125}$I-fibrin decomposition products isolated in the supernatant were measured with a $\gamma$-counter. The sample number was 3 in each group.

As shown in Table 3, it is clear that when the plasma having lowered $\alpha_2$-PI activity was used, lysis of the fibrin clots was accelerated by administration of the test compound, as compared to the plasma prior to the administration.

Table 3

| Rate of Lysis of Fibrin Clot (%) | | |
|---|---|---|
| Urokinase Activity | Prior to Administration | 4 Days After Administration |
| 0 U | 18.1 | 21.5 |
| 15 U | 37.4 | 63.8 |
| 30 U | 70.0 | 93.7 |

Test Example 4 <u>Thrombolytic test in vitro</u>

Thrombolytic activity-inducing ability in the established vascular endothelial cell culture system was examined using CPAE (calf pulmonary artery endothelia).

EPAE cells were purchased from Dainippon Pharmaceutical Co., Ltd., Department of Laboratory Products. CPAE cells were subcultured in 10% FCS-Eagle MEM medium charged in a culture flask of 25 $cm^2$. From the cell suspension fractionated upon subculture, 0.1 ml was transferred into a test tube with a sterilized pipette. Then, the cell suspension was diluted to 10 fold with 0.9 ml of Trypan Blue solution and a cell count was counted with a cell counter. After diluting with 10% FCS-Eagle MEM medium so as to have a cell count of 2 x $10^5$ cells/ml, 100 µl each/well, namely, 2 x $10^4$ cells/well, of the dilution was dispensed in a 96 well microtiter plate (manufactured by Corning) with a micropipette. The plate was incubated at 37°C in 5% $CO_2$.

The compound used according to the present invention was dissolved in medium in 0.2 mg/ml; the compound that was insoluble in the medium was dissolved in less than 1% of DMSO and the resulting solution was aseptically filtered through a filter; 5 µl of the solution was added to the well with a sterilized micropipette 24 hours after onset of the incubation. After culturing at 37°C for 72 hours in 5% $CO_2$, the collected culture supernatant was provided for measurement.

The measurement was performed as follows. Plasminogen, 5 µl, was charged in a well of fibrin plate (manufactured by Kitazato institute), which was allowed to stand until diffusion was completed. After diffusion, 5 µl of the supernatant was charged and then put in a carbon dioxide gas incubator of 37°C. Four hours after, evaluation was made by formation of a transparent lysis circle by fibrinolysis. In this case, it was confirmed that a transparent circle was simultaneously formed by fibrinolysis in a well charged with t-PA as a positive control. A diameter of the transparent circle was 9 to 10 mm.

Also in the case of the supernatant added with the compound used according to the present invention, a transparent circle due to fibrinolysis showing a diameter of 4.5 to 8.5 mm was noted. However, in the case of the supernatant added with no compound used according to the present invention (control), no change was noted. In the case of forming a circle of 4 mm or more due to fibrinolysis, it was judged that thrombolytic ability of CPAE cells was induced.

After the fibrinolytic activity was checked over, the well was fixed with 2.5% of glutaraldehyde in a final concentration. Then, the solution was discarded and the system was washed with PBS. After washing, moisture was removed. After staining with 100 µl of 0.1% crystal violet and allowing stand for 2 to 3 minutes, the system was washed with running water. After an excess of the staining solution was washed out, moisture was removed and the dye bound to the cells was eluted by 100 µl of methanol. Using multiscanning (Titertech), measurements were performed at a wavelength of 580 nm in accordance with the ABS method and the matrix method to confirm that the cells were not injured.

A diameter (mm) of the circle due to fibrinolysis is shown in Table 4. It is clearly seen that the compounds used

according to the present invention showed the thrombolytic activity.

Table 4

| Compound No. | Diameter (mm) | Compound No. | Diameter (mm) |
|---|---|---|---|
| 2 | 7.9 | 25 | 4.1 |
| 8 | 6.3 | 26 | 5.7 |
| 9 | 8.2 | 27 | 8.5 |
| 10 | 4.5 | 28 | 8.3 |
| 12 | 5.9 | 29 | 7.1 |
| 14 | 5.9 | 30 | 7.6 |
| 15 | 6.3 | 31 | 7.9 |
| 17 | 7.5 | 32 | 7.5 |
| 18 | 6.8 | 33 | 7.8 |
| 19 | 5.8 | 34 | 7.6 |
| 20 | 8.0 | 35 | 7.8 |
| 21 | 5.6 | 36 | 7.6 |
| 22 | 6.5 | 37 | 6.9 |
| 23 | 8.4 | 38 | 6.9 |
| 24 | 7.6 | 2a | 7.5 |

Test Example 5 Thrombolytic activity in vitro

It has been clarified that the compounds used according to the present invention are capable of inducing fibrinolytic activity in CPAE cells. In order to verify by what substance in addition to the $\alpha_2$-PI lowering activity, this fibrinolytic activity is induced, analysis was made on the culture solution added with Compound No. 2 out of the compounds used according to the present invention, by means of fibrin autography.

By SDS-polyacrylamide gel using 10% gel, this culture solution and t-PA (tissue plasminogen activator) and urokinase were subjected to electrophoresis. After the electrophoresis, the gel was treated with 2.5% Triton X-100, which was inoculated on agar plate added with fibrinogen, thrombin and plasminogen. A place of fibrin which caused lysis was confirmed in an incubator of 37°C in 5% $CO_2$.

As a result, great fibrinolysis was noted in the culture supernatant added with the compound used according to the present invention, at the position of molecular weight of urokinase type plasminogen activator. It was made clear that the compound used according to the present invention strongly induced production of urokinase type plasminogen activator in CPAE.

Test on acute toxicity:

Four (4) ddY strain male mice of 6 week age were used for each group of the test sample.

Method:

In intravenous administration, each sample was dissolved in 0.9% physiological saline and the solution was administered through the tail vein. In intraperitoneal administration, each sample was suspended in 0.5% CMC-physiological saline and 0.1 ml of the suspension per 10 mg of mouse body weight was intraperitoneally administered. In oral administration, each sample was suspended in 0.5% CMC-physiological saline and 0.2 ml of the suspension per 10 mg of mouse body weight was orally administered.

Observation was made immediately after the administration. After observation for 1 week after the administration, the mice was sacrificed with chloroform and subjected to autopsy.

$LD_{50}$ of each sample is summarized in the following table. Safety of the compounds used according to the present invention are clearly shown.

| Intravenous administration: | |
|---|---|
| (Compound No.) | $LD_{50}$ (mg/kg) |
| 1 | 3235 |
| 2 | 5091 |

| Intraperitoneal administration: | |
|---|---|
| (Compound No.) | $LD_{50}$ (mg/kg) |
| 1 | 5,000 |
| 2 | 10,000 |

| Oral administration: | |
|---|---|
| (Compound No.) | $LD_{50}$ (mg/kg) |
| 1 | 7,500 |
| 2 | 10,000 |

Example 1

Per 1 tablet, the following compounds were added to the compound (Compound No. 2) used according to the present invention and tablets were obtained in a conventional manner.

| Per tablet (in 300 mg) | |
|---|---|
| Compound used in the present invention (Compound No. 2) | 200 mg |
| Lactose | 50 mg |
| Corn starch | 20 mg |
| Low substitution degree hydroxypropyl cellulose | 15 mg |
| Hydroxypropyl cellulose | 5 mg |
| Magnesium stearate | 10 mg |
| | 300 mg |

Example 2

Per 1 ampoule, the following compounds were added to the compound (Compound No. 2) used according to the

present invention and ampoules for injection were obtained in a conventional manner.

| Per ampoule (in 10 ml) | |
|---|---|
| Compound used in the present invention (Compound No. 2) | 200 mg |
| Sodium chloride | 90 mg |
| Distilled water for injection | q.s. |
| | 10 ml |

## Claims

1. Use of a composition comprising as the main ingredient a compound selected from the group consisting of (1) through (4) below:

   (1) a compound represented by the following general formula [II] and a physiologically acceptable acid addition salt thereof as well as a quaternary salt thereof;
   (2) nojirimycin and a physiologically acceptable acid addition salt thereof;
   (3) 1,4-bis(3-moranolino-1-propenyl)benzene and a physiologically acceptable acid addition salt thereof; and
   (4) castanospermine;

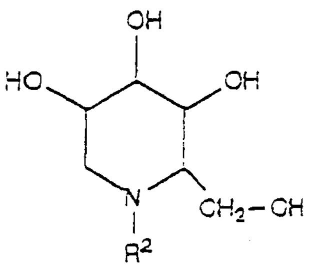

[II]

   wherein $R^2$ represents a hydrogen atom or an amino, methoxyethyl, methoxyethoxyethyl, 2-hydroxyhexadecyl, 3-carbamoylpropyl, undecyl, 2-hydroxytetradecyl or 3-carboxy-2-propenyl group, or an alkyl, carboxyalkyl, alkyloxycarbonylalkyl, hydroxyalkyl, cycloalkylalkyl, arylalkyl, aryloxyalkyl, alkenyl, hydroxyalkenyl, arylalkenyl, aryloxyalkenyl or alkylcarbamoylalkyl; 3-fluoro-4-methylphenylbutyl, 2-hydroxy-3-phenoxypropyl, 3-o-chlorophenoxypropyl, p-ethoxycarbonylphenoxyethyl, 2-hydroxy-3-tolyloxypropyl, 2-hydroxy-3-p-methoryphenoxypropyl, 2-hydroxy-3-p-chlorophenoxypropyl, 3-(m-methoxyethoxyphenyl)-2-butenyl, γ-methyl-4-bromocinnamyl, p-ethoxycinnamyl, p-isopropyloxycinnamyl, γ-methyl-m-methylcinnamyl, 4-(m-methoxyphenyl)-3-pentenyl, 4,4-diphenyl-3-butenyl, 3-(p-chlorophenyl)-2-butenyl, 3-(p-methylphenyl)-2-butenyl, 4-(p-chlorophenyl)-3-pentenyl, 4-(m-chlorophenyl)-3-pentenyl, 4-(o-chlorophenyl)-3-pentenyl, 4-(p-phenoxyphenyl)-3-pentenyl, 4-(p-ethoxyphenyl)-3-pentenyl, m-methoxycinnamyl, 3-(m-chlorophenyl)-2-butenyl, 4-(p-chlorophenyl)-3-butenyl, p-carboxycinnamyl, m-trimethylammonioethoxycinnamyl, p-trimethylammonioethoxycinnamyl; wherein the cycloalkyl of the cycloalkylalkyl is cyclopropyl, cyclobutyl or cyclopentyl; the aryl is phenyl or naphthyl; the alkyl groups contain up to 10 carbon atoms,

   for the manufacture of a medicament for the treatment of myocardial infarction, cerebral infarction or for the prevention of postoperative thrombus or thrombosis.

2. Use of a compound represented by the general formula [II] as claimed in claim 1, wherein $R^2$ is selected from the group consisting of benzyl, phenylethyl, phenylpropyl, phenylbutyl, phenylpentyl, 3-fluoro-4-methylphenylbutyl, naphthylmethyl, naphthylethyl, naphthylpropyl or naphthylbutyl.

3. Use of a compound represented by the general formula [II] as claimed in claim 1, wherein $R^2$ is selected from the group consisting of phenoxymethyl, phenoxyethyl, phenoxypropyl, phenoxybutyl, 2-hydroxy-3-phenoxypropyl, 3-o-chlorophenoxypropyl, p-ethoxycarbonylphenoxyethyl, 2-hydroxy-3-tolyloxypropyl, 2-hydroxy-3-p-methoxyphenoxypropyl, 2-hydroxy-3-p-chlorophenoxypropyl, naphthyloxymethyl, naphthyloxypropyl or naphthyloxybutyl.

4. Use of a compound represented by the general formula [II] as claimed in claim 1, wherein $R^2$ is selected from the group consisting of cinnamyl, 3-(m-methoxyethoxyphenyl)-2-butenyl, 4-phenyl-3-butenyl, β-methylcinnamyl, γ-methyl-4-bromocinnamyl, p-ethoxycinnamyl, p-isopropyloxycinnamyl, γ-methyl-m-methylcinnamyl, 4-(m-methoxyphenyl)-3-pentenyl, 4,4-diphenyl-3-butenyl, 3-(p-chlorophenyl)-2-butenyl, 3-(p-methylphenyl)-2-butenyl, 4-(p-chlorophenyl)-3-pentenyl, 4-(m-chlorophenyl)-3-pentenyl, 4-(o-chlorophenyl)-3-pentenyl, 4-(p-phenoxyphenyl)-3-pentenyl, 4-(p-ethoxyphenyl)-3-pentenyl, m-methoxycinnamyl, 3-(m-chlorophenyl)-2-butenyl, 4-(p-chlorophenyl)-3-butenyl, p-carboxycinnamyl, m-trimethylammonioethoxycinnamyl, p-trimethylammonioethoxycinnamyl, phenylvinyl, phenylpropenyl, phenylbutenyl, naphthylvinyl, naphthylpropenyl or naphthylbutenyl.

5. Use of a compound represented by the general formula [II] as claimed in claim 1, wherein $R^2$ represents an alkenyl selected from vinyl, propenyl, butenyl, geranyl, farnesyl or isoprenyl.

6. Use of a compound represented by the general formula [II] as claimed in claim 1, wherein $R^2$ is selected from the group consisting of benzyl, phenylpropyl, phenylbutyl, phenylpentyl or 3-fluoro-4-methylphenylbutyl.

7. Use of a compound represented by the general formula [II] as claimed in claim 1, wherein $R^2$ is selected from the group consisting of phenoxyethyl, phenoxypropyl, 2-hydroxy-3-phenoxypropyl, 3-o-chlorophenoxypropyl or p-ethoxycarbonylphenoxyethyl.

8. Use of a compound represented by the general formula [II] as claimed in claim 1, wherein $R^2$ is selected from the group consisting of cinnamyl, 3-(m-methoxyethoxyphenyl)-2-butenyl, 4-phenyl-3-butenyl, β-methylcinnamyl, γ-methyl-4-bromocinnamyl, p-ethoxycinnamyl, p-isopropyloxycinnamyl, γ-methyl-m-methylcinnamyl, 4-(m-methoxyphenyl)-3-pentenyl.

9. Use of the compound represented by the general formula [II] as claimed in claim 1, wherein the compound is N-methylmoranoline.

**Patentansprüche**

1. Verwendung einer Zusammensetzung enthaltend als Hauptbestandteil eine aus (1) bis (4) ausgewählte nachfolgende Verbindung:

(1) eine durch die allgemeine Formel [II] dargestellte Verbindung und deren physiologisch annehmbares Säureadditionssalz sowie deren quaternäres Salz,
(2) Nojirimycin und dessen physiologisch annehmbaren Säureadditionssalz,
(3) 1,4-Bis-(3-moranolino-1-propenyl)benzol und dessen physiologisch annehmbares Säureadditionssalz, und
(4) Castanospermin;

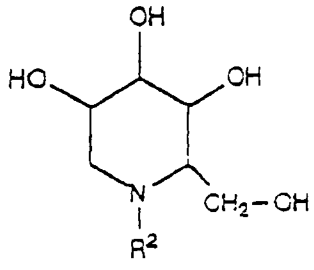

[II]

worin $R^2$ ein Wasserstoffatom oder eine Amino-, Methoxyethyl-, Methoxyethoxyethyl-, 2-Hydroxyhexadecyl-, 3-Carbamoylpropyl-, Undecyl-, 2-Hydroxytetradecyl- oder 3-Carboxy-2-propenyl-Gruppe, oder ein Alkyl, Carboxyalkyl, Alkyloxycarbonylalkyl, Hydroxyalkyl, Cycloalkylalkyl, Arylalkyl, Aryloxyalkyl, Alkenyl, Hydroxyalkenyl, Arylalkenyl, Aryloxyalkenyl oder Alkylcarbarnoylalkyl; 3-Fluor-4-methylphenylbutyl, 2-Hydroxy-3-phenoxypropyl, 3-o-Chlorphenoxypropyl, p-Ethoxycarbonylphenoxyethyl, 2-Hydroxy-3-tolyloxypropyl, 2-Hydroxy-3-p-methoxyphenoxypropyl, 2-Hydroxy-3-p-chlorphenoxypropyl, 3-(m-Methoxyethoxyphenyl)-2-butenyl, γ-Methyl-4-bromcinnamyl, p-Ethoxycinnamyl, p-Isopropyloxycinnamyl, γ-Methyl-m-methylcinnamyl, 4-(m-Methoxyphenyl)-3-pentyl, 4,4-Diphenyl-3-butenyl, 3-(p-Chlorphenyl)-2-butenyl, 3-(p-Methylphenyl)-2-butenyl, 4-(p-Chlorphenyl)-3-pentenyl, 4-(o-Chlorphenyl)-3-pentenyl, 4-(m-Chlorphenyl)-3-pentenyl, 4-(p-Phenoxyphenyl)-3-pentenyl, 4-(p-Ethoxyphenyl)-3-pentenyl, m-Methoxycinnamyl, 3-(m-Chlorphenyl)-2-butenyl, 4-(p-Chlorphenyl)-3-butenyl, p-Carboxycinnamyl, m-Trimethylammonioethoxycinnamyl, p-Trimethylammonioethorycinnamyl; worin das Cycloalkyl des Cycloalkylalkyls Cyclopropyl, Cyclobutyl oder Cyclopentyl; das Aryl, Phenyl oder Naphthyl; die Alkylgruppen bis zu 10 Kohlenstoffatome enthalten, darstellt,

zur Herstellung eines Medikaments zur Behandlung des Myocardinfarkts, Gehirninfarkts oder für die Prävention von postoperativem(n) Thrombus oder Thrombosen.

2. Verwendung einer durch die allgemeine Formel [II] dargestellten Verbindung nach Anspruch 1, worin $R^2$ aus Benzyl, Phenylethyl, Phenylpropyl, Phenylbutyl, Phenylpentyl, 3-Fluor-4-methylphenylbutyl, Naphthylmethyl, Naphthylethyl, Naphthylpropyl oder Naphthylbutyl ausgewählt ist.

3. Verwendung einer durch die allgemeine Formel [II] dargestellten Verbindung nach Anspruch 1, worin $R^2$ aus Phenoxymethyl, Phenoxyethyl, Phenoxypropyl, Phenoxybutyl, 2-Hydroxy-3-phenoxypropyl, 3-o-Chlorphenoxypropyl, p-Ethoxycarbonylphenoxyethyl, 2-Hydroxy-3-tolyloxypropyl, 2-Hydroxy-3-p-methoxyphenoxypropyl, 2-Hydroxy-3-p-chlorphenoxypropyl, Naphthyloxymethyl, Naphthyloxypropyl oder Naphthyloxybutyl ausgewählt ist.

4. Verwendung einer durch die allgemeine Formel [II] dargestellten Verbindung nach Anspruch 1, worin $R^2$ aus Cinnamyl, 3-(m-Methoxyethoxyphenyl)-2-butenyl, 4-Phenyl-3-butenyl, β-Methylcinnamyl, γ-Methyl-4-bromcinnamyl, p-Ethoxycinnamyl, p-Isopropyloxycinnamyl, γ-Methyl-m-methylcinnamyl, 4-(m-Methoxyphenyl)-3-pentenyl, 4,4-Diphenyl-3-butenyl, 3-(p-Chlorphenyl)-2-butenyl, 3-(p-Methylphenyl)-2-butenyl, 4-(p-Chlorphenyl)-3-pentenyl, 4-(m-Chlorphenyl)-3-pentenyl, 4-(o-Chlorphenyl)-3-pentenyl, 4-(p-Phenoxyphenyl)-3-pentenyl, 4-(p-Ethoxyphenyl)-3-pentenyl, m-Methoxycinnamyl, 3-(m-Chlorphenyl)-2-butenyl, 4-(p-Chlorphenyl)-3-butenyl, p-Carboxycinnamyl, m-Trimethylammonioethoxycinnamyl, p-Trimethylammonioethoxycinnamyl, Phenylvinyl, Phenylpropenyl, Phenylbutenyl, Naphthylvinyl, Naphthylpropenyl oder Naphthylbutenyl ausgewählt ist.

5. Verwendung einer durch die allgemeine Formel [II] dargestellten Verbindung nach Anspruch 1, worin $R^2$ ein aus Vinyl, Propenyl, Butenyl, Geranyl, Farnesyl oder Isoprenyl ausgewähltes Alkenyl darstellt.

6. Verwendung einer durch die allgemeine Formel [II] dargestellten Verbindung nach Anspruch 1, worin $R^2$ aus Benzyl, Phenylpropyl, Phenylbutyl, Phenylpentyl oder 3-Fluor-4-methylphenylbutyl ausgewählt ist.

7. Verwendung einer durch die allgemeine Formel [II] dargestellten Verbindung nach Anspruch 1, worin $R^2$ aus Phenoxyethyl, Phenoxypropyl, 2-Hydroxy-3-phenoxypropyl, 3-o-Chlorphenoxypropyl oder p-Ethoxycarbonylphenoxyethyl ausgewählt ist.

8. Verwendung einer durch die allgemeine Formel [II] dargestellten Verbindung nach Anspruch 1, worin $R^2$ aus Cinnamyl, 3-(m-Methoxyethoxyphenyl)-2-butenyl, 4-Phenyl-3-butenyl, β-Methylcinnamyl, γ-Methyl-4-bromcinnamyl, p-Ethoxycinnamyl, p-Isopropyloxycinnamyl, γ-Methyl-m-methylcinnamyl, 4-(m-Methoxyphenyl)-3-pentenyl ausgewählt ist.

9. Verwendung einer durch die allgemeine Formel [II] dargestellten Verbindung nach Anspruch 1, worin die Verbindung N-Methylmoranolin ist.

## Revendications

1. Utilisation d'une composition comprenant, en tant qu'ingrédient principal, un composé choisi dans le groupe constitué de (1) à (4) ci-dessous :

(1) un composé représenté par la formule générale [II] suivante et un sel, physiologiquement acceptable, d'addition de celui-ci avec un acide, ainsi qu'un sel quaternaire de celui-ci ;
(2) la nojirimycine et un sel, physiologiquement acceptable, d'addition de celle-ci avec un acide ;
(3) le 1,4-vis(3-moranolino-1-propényl)benzène et un sel, physiologiquement acceptable, d'addition de celui-ci

avec un acide ; et

(4) la castanospermine ;

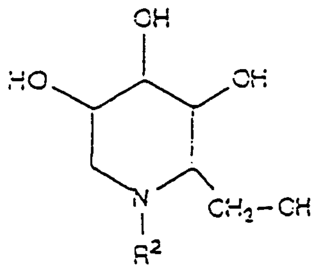

[II]

dans laquelle $R^2$ représente un atome d'hydrogène ou un groupe amino, méthoxyéthyle, méthoxyéthoxyéthyle, 2-hydroxyhexadécyle, 3-carbamoylpropyle, undécyle, 2-hydroxytétradécyle ou 3-carboxy-2-propényle, ou un groupe alkyle, carboxyalkyle, alkyloxycarbonylalkyle, hydroxyalkyle, cycloalkylalkyle, arylalkyle, aryloxyalkyle, alcényle, hydroxyalcényle, arylalcényle, aryloxyalcényle ou alkylcarbamoylalkyle ; 3-fluoro-4-méthylphénylbutyle, 2-hydroxy-3-phénoxypropyle, 3-o-chlorophénoxypropyle, p-éthoxycarbonylphénoxyéthyle, 2-hydroxy-3-tolyloxypropyle, 2-hydroxy-3-p-méthoxyphénoxypropyle, 2-hydroxy-3-p-chlorophénoxypropyle, 3-(m-méthoxyéthoxyphényl)-2-butényle, γ-méthyl-4-bromocinnamyle, p-éthoxycinnamyle, p-isopropyloxycinnamyle, γ-méthyl-m-méthylcinnamyle, 4-(m-méthoxyphényl)-3-pentényle, 4,4-diphényl-3-butényle, 3-(p-chlorophényl)-2-butényle, 3-(p-méthylphényl)-2-butényle, 4-(p-chlorophényl)-3-pentényle, 4-(m-chlorophényl)-3-pentényle, 4-(o-chlorophényl)-3-pentényle, 4-(p-phénoxyphényl)-3-pentényle, 4-(p-éthoxyphényl)-3-pentényle, m-méthoxycinnamyle, 3-(m-chlorophényl)-2-butényle, 4-(p-chlorophényl)-3-butényle, p-carboxycinnamyle, m-triméthylammonioéthoxycinnamyle, p-triméthylammonioéthoxycinnamyle ; où le groupe cycloalkyle du groupe cycloalkylalkyle est le groupe cyclopropyle, cyclobutyle ou cyclopentyle ; le groupe aryle est le groupe phényle ou naphtyle ; les groupes alkyles contiennent jusqu'à 10 atomes de carbone,

pour la fabrication d'un médicament pour le traitement d'un infarctus du myocarde, d'un infarctus cérébral ou pour la prévention d'un thrombus ou d'une thrombose postopératoire.

2. Utilisation d'un composé représenté par la formule générale [II] selon la revendication 1, dans laquelle $R^2$ est choisi dans le groupe constitué des groupes benzyle, phényléthyle, phénylpropyle, phénylbutyle, phénylpentyle, 3-fluoro-4-méthylphénylbutyle, naphtylméthyle, naphtyléthyle, naphtylpropyle ou naphtylbutyle.

3. Utilisation d'un composé représenté par la formule générale [II] selon la revendication 1, dans laquelle $R^2$ est choisi dans le groupe constitué des groupes phénoxyméthyle, phénoxyéthyle, phénoxypropyle, phénoxybutyle, 2-hydroxy-3-phénoxypropyle, 3-o-chlorophénoxypropyle, p-éthoxycarbonylphénoxyéthyle, 2-hydroxy-3-tolyloxypropyle, 2-hydroxy-3-p-méthoxyphénoxypropyle, 2-hydroxy-3-p-chlorophénoxypropyle, naphtyloxyméthyle, naphtyloxypropyle ou naphtyloxybutyle.

4. Utilisation d'un composé représenté par la formule générale [II] selon la revendication 1, dans laquelle, $R^2$ est choisi dans le groupe constitué des groupes cinnamyle, 3-(m-méthoxyéthoxyphényl)-2-butényle, 4-phényl-3-butényle, β-méthylcinnamyle, γ-méthyl-4-bromocinnamyle, p-éthoxycinnamyle, p-isopropyloxycinnamyle, γ-méthyl-m-méthylcinnamyle, 4-(m-méthoxyphényl)-3-pentényle, 4,4-diphényl-3-butényle, 3-(p-chlorophényl)-2-butényle, 3-(p-méthylphényl)-2-butényle, 4-(p-chlorophényl)-3-pentényle, 4-(m-chlorophényl)-3-pentényle, 4-(o-chlorophényl)-3-pentényle, 4-(p-phénoxyphényl)-3-pentényle, 4-(p-éthoxyphényl)-3-pentényle, m-méthoxycinnamyle, 3-(m-chlorophényl)-2-butényle, 4-(p-chlorophényl)-3-butényle, p-carboxycinnamyle, m-triméthylammonioéthoxycinnamyle, p-triméthylammonioéthoxycinnamyle, phénylvinyle, phénylpropényle, phénylbutényle, naphtylvinyle, naphtylpropényle ou naphtylbutényle.

5. Utilisation d'un composé représenté par la formule générale [II] selon la revendication 1, dans laquelle $R^2$ représente un groupe alcényle choisi parmi les groupes vinyle, propényle, butényle, géranyle, farnésyle ou isoprényle.

6. Utilisation d'un composé représenté par la formule générale [II] selon la revendication 1, dans laquelle $R^2$ est choisi dans le groupe constitué des groupes benzyle, phénylpropyle, phénylbutyle, phénylpentyle ou 3-fluoro-4-méthylphénylbutyle.

7. Utilisation d'un composé représenté par la formule générale [II] selon la revendication 1, dans laquelle $R^2$ est choisi dans le groupe constitué des groupes phénoxyéthyle, phénoxypropyle, 2-hydroxy-3-phénoxypropyle, 3-o-chlorophénoxypropyle ou p-éthoxycarbonylphénoxyéthyle.

8. Utilisation d'un composé représenté par la formule générale [II] selon la revendication 1, dans laquelle $R^2$ est choisi dans le groupe constitué des groupes cinnamyle, 3-(m-méthoxyéthoxyphényl)-2-butényle, 4-phényl-3-butényle, β-méthylcinnamyle, γ-méthyl-4-bromocinnamyle, p-éthoxycinnamyle, p-isopropyloxycinnamyle, γ-méthyl-m-méthylcinnamyle, 4-(m-méthoxyphényl)-3-pentényle.

9. Utilisation d'un composé représenté par la formule générale [II] selon la revendication 1, dans laquelle le composé est la N-méthylmoranoline.